# EUROPEAN PATENT APPLICATION

(11) **EP 1 754 535 A1**
(43) Date of publication of application: **21.02.2007**
(21) Application number: 06021462.4
(22) Date of filing: 08.07.2002
(51) Int. Cl.: B01J 35/02, B01J 23/72, B01J 23/89, C07C 7/167

(54) **Process for hydrogenating acetylenes**

(62) Divisional of application: 02748159.7
(71) Applicant: UOP LLC, Des Plaines, IL 60017-5017 (US)
(72) Inventor: Abrevaya, Hayim, Des Plaines IL 60017-5017 (US); Jan, Deng-Yang, Des Plaines IL 60017-5017 (US); Streigleder, Karl, Z., Des Plaines IL 60017-5017 (US)
(74) Representative: Chung, Hsu Min

(57) **Abstract**

A process for selectively hydrogenating C4-acetylenes in a liquid hydrocarbon stream containing largely butadiene comprising contacting hydrogen and the hydrocarbon stream with a catalytic composite comprising an inorganic oxide support having dispersed thereon finely divided copper metal and an activator metal selected from the group consisting of nickel, cobalt, platinum, palladium, manganese, and a combination thereof said catalytic composite having an average effective diameter of up to 800 µm (1/32 inch).

## Description

### BACKGROUND OF THE INVENTION

Processes for the selective hydrogenation of C₄-acetylenes in the presence of butadiene with the added benefit of extended catalyst stability are sought.

Butadiene is an important starting material for the production of high molecular weight polymers and is used extensively to form synthetic rubber including styrene-butadiene rubber, nitrile-butadiene rubber, buna-S rubber, and trans-polybutadiene rubber, and adiponitrile and styrene butadiene latex in paints. Butadiene is usually a by-product from steam cracking naphtha. However, the product butadiene regularly contains impurities that must be removed before the butadiene may be used as a starting material. The principal impurities are acetylenes including ethylacetylene, methylacetylene and vinylacetylene. Historically, two approaches have been used to remove the acetylenes: extractive distillation using a solvent to selectively absorb the acetylenes, or selective hydrogenation of the acetylenes.

In using selective hydrogenation, copper-containing catalytic composites have been successful. US-A-4,493,906 discloses copper-containing catalytic composites for selective hydrogenation of acetylenes in the form of 1600 µm (1/16 inch) extrudates. US-A-4,440,956 discloses the catalysts as 1/8 inch (3mm) pellets. US-A-3,912,789, and US-A-3,218,268 disclose the catalysts as 3/16 inch tablets. US-A-3,751,508 discloses the catalysts as 3 mm tablets (1/8 inch tablet). A microsphere catalyst has been discovered to have much improved stability and selectivity versus similar catalysts having particles of 1/16 inch (1600 µm) diameter. Improved catalyst performance has also been discovered with a copper-containing catalytic composite where at least 70 weight percent of the copper and optionally one or more activator metals are dispersed on the outer 200 µm of the catalyst support. It is most preferred that the catalyst composite particles also have an average diameter of 800 µm (1/32 inch) or less.

### SUMMARY OF THE INVENTION

The present invention is an improved process for selectively hydrogenating C₄-acetylenes in a liquid hydrocarbon stream containing largely butadiene with the benefit of increased catalyst stability. Hydrogen and the hydrocarbon stream contact a catalytic composite comprising an inorganic oxide support having dispersed thereon finely divided copper metal and optionally an activator metal selected from the group consisting of nickel, cobalt, platinum, palladium, manganese, and a combination. At least 70 weight percent of the copper metal and the activator metal are dispersed on the outer 200 µm layer of the support. In a specific embodiment of the invention, at least 80 weight percent of the copper metal is dispersed on the outer 200 µm layer of the support. In another specific embodiment of the invention, hydrogen and the hydrocarbon stream contacted a spherical catalytic composite having an average diameter of 800 µm (1/32 inch) or less. The catalytic composite comprises an inorganic oxide support having dispersed thereon finely divided copper metal and optionally an activator metal selected from the group consisting of nickel, cobalt, platinum, palladium, manganese.

### BRIEF DESCRIPTION OF THE DRAWINGS

The figures depict results of accelerated stability experiments conducted in Example 3 by comparing the catalyst of the present invention with three reference catalysts over time. FIG. 1 and FIG. 2 show the weight percent conversion of vinyl acetylene and total acetylenes respectively. FIG. 3 shows 1,3-butadiene retention over time. FIG. 4 shows weight percent hydrogen conversion. FIG 5. shows the hydrogen:acetylene usage ratio (moles hydrogen consumed divided by moles acetylenes consumed). FIG. 6 shows selectivity to polymeric byproducts.

### DETAILED DESCRIPTION OF THE INVENTION

The invention is a process for selectively hydrogenating C₄-acetylenes in the presence of large amounts of butadiene by contacting the hydrocarbons with a supported catalytic composite. In one embodiment at least 50 weight percent of the active catalytic agents, preferably at least 70 weight percent, more preferably at least 80 weight percent, and most preferably at least 88 weight percent, are located on the outer 200 µm layer of the support. In another embodiment of the invention the support is spherical and has an average diameter of less than 800 µm (1/32 inch). The invention further reduces the production of undesired high molecular weight polymerized byproducts thereby extending the stability and enhancing the selectivity of the catalyst. The term "C₄-acetylenes" as used herein is meant to include vinylacetylene, ethylacetylene, and methylacetylene. The vinylacetylene is hydrogenated to 1,3-butadiene, the ethylacetylene is hydrogenated to 1-butene, and the methylacetylene is hydrogenated to propylene.

The C₄-acetylenes are typically formed as a byproduct in butadiene production and must be removed before the butadiene can be further processed. The acetylenes may be in a concentration ranging from 0.5 to 3 weight percent, or higher, of the product liquid hydrocarbon stream from a butadiene production reactor. The liquid hydrocarbon stream generally contains butadiene (40-50 weight percent), butenes (40-50 weight percent), butanes (5-10 weight percent) and C₄-acetylenes. Propane and C₃-acetylenes are also present in minor quantities. In a typical treating process, hydrogen and the hydrocarbon stream enter a fixed bed reactor. Various methods of introducing hydrogen to the reactor are known and any such method is suitable for use in this invention. The preferred method admixes the hydrocarbon stream with a stoichiometric amount of hydrogen and then introduces the mixture to a fixed bed reactor.

The fixed bed reactor contains a catalytic composite effective to catalyze the selective hydrogenation of the acetylenes. The catalytic composite must be "selective" to the acetylenes so as to minimize hydrogenation of the desired butadiene. The catalytic composite contains finely divided copper metal and one or more activator metals which are bound to a support. The activator metals are those which are normally introduced in the form of salts and whose oxides are reducible by hydrogen. Suitable activator metals include nickel, cobalt, manganese, platinum, palladium, or a combination thereof. The most preferred activator metal is nickel. The copper is present in an amount ranging from 5 to 15 weight percent of the whole finished catalytic composite in the oxidized form and the activator metal is present in an amount ranging from 0.1 to 1 weight percent of the whole finished catalytic composite in the oxidized form.

The support may be refractory inorganic oxide materials such as silica, alumina, carbon, titania, magnesia, zirconia, clays, zeolites, and a combination thereof. The aluminas for supports include gamma, theta, delta, and alpha alumina with gamma and theta alumina being preferred. Suitable zeolites include faujasites, zeolite Beta, L-zeolite, ZSM-5, ZSM-8, ZSM-11, ZSM-12, and ZSM-35.

The support may be of any suitable size and shape including spherical and extruded supports. The extruded support is prepared as commonly known in the art. An extrudate is preferably cylindrical with a 800 µm (1/32 inch) diameter. The support may also be a shaped support such as a trilobe, quadrulobe, irregular shaped particles, pellets, or hollow tube which preferably posses a maximum diffusion path of 800 µm (1/32 inch) or less. The support may also be spherical with typical sphere sizes used in process such as these include 1600 µm (1/16 inch) and 3200 µm (1/8 inch). A preferred spherical support is of a "microsphere" size, which includes spheres of support material nominally having a diameter of 800 µm (1/32 inch) or less. The spheres are preferably produced by commonly known oil-dropping techniques such as described in United States Patent No. 2,620,314, which is incorporated by reference. The oil drop method comprises forming an aluminum hydrosol, preferably by reacting aluminum metal with hydrochloric acid; combining the hydrosol with a suitable gelling agent, e.g., hexamethylenetetramine; and dropping the resultant mixture into and oil bath maintained at elevated temperatures. The mixture droplets remain in the oil bath until they set and form hydrogel spheres. The spheres are then continuously withdrawn from the oil bath and typically subjected to specific aging and drying treatments in oil and ammoniacal solutions to further improve their physical characteristics. Aged gel spheres undergo washing at 70°C to 100°C, drying at a temperature of 65°C to 260°C, and calcining for 1 to 20 hours at a temperature of 455°C to 705°C. This treatment converts the hydrogel to the corresponding crystalline gamma-alumina. If theta alumina is desired then the hydrogel spheres are calcined at a temperature of 950°C to 1200°C.

While microspheres are preferred, a variety of support shapes are suitable, as discussed above. It is preferred that the support, whether spherical or not, have an effective diameter of 800 µm (1/32 inch) or less. For a non-spherical support, effective diameter is defined as the diameter of the shaped article would have if it were molded into a sphere.

The catalytic metal copper, and the activator metal(s) may be dispersed onto the support by means well known in the art such as impregnation, coprecipitation, cogellation or ion exchange. The preferred method of incorporating the metal copper and the activator metals is impregnation of the support with a solution containing one or more decomposable compound of the desired metal(s) followed by calcination. Illustrative of the decomposable compounds which can be used are: copper nitrate, copper acetate, copper acetylacetonate, nickel nitrate, nickel carbonate, nickel acetate, nickel acetylacetonate, manganese nitrate, manganese acetate, manganese acetylacetonate, manganese carbonate, manganese carbonyl, cobalt nitrate, cobalt acetate, cobalt acetylacetonate, cobalt carbonate, chloroplatinic acid, platinum tetrachloride, palladic acid, palladium chloride, and palladium nitrate.

Suitable impregnation techniques include dip, evaporative and vacuum impregnation. A preferred impregnation method uses of a steam-jacketed rotary evaporator. The desired support is immersed in an impregnating solution containing the desired metal(s) in the drier and the support is tumbled therein by the rotary motion of the drier. Evaporation of the solution in contact with the tumbling support is expedited by applying the steam to the drier jacket. The resultant catalytic composite is dried and then calcined.

The use of a microspherical catalytic composite in this invention has several advantages over previously disclosed catalysts. A portion of the acetylenes present in the hydrocarbon stream will tend to polymerize and form high molecular weight undesirable byproducts, see, Sarkany, A.; Weiss, A. H.; Szilagyi, T.; Sandor P.; Guczi L. Applied Catalysis 1984, 12, 373-379. Furthermore, much of the polymerization occurs within the pores of the catalytic composite with the polymerized products remaining trapped within the pores and decreasing the activity of the catalyst. As the activity of the catalyst declines, the selective nature of the hydrogenation decreases and the amount of hydrogenation of butadiene relative to the amount of hydrogenation of acetylenes increases. Through using a microspherical catalytic composite, the residence time of the acetylene within the catalyst before being hydrogenated is reduced thereby decreasing the opportunity for the acetylene to polymerize. In other words, the diffusion path length of the acetylene through the composite is reduced allowing for more rapid hydrogenation and less acetylene is available for polymerization. Reducing the amount of acetylene polymerization results in increased catalytic composite stability and enhanced selectivity. With increased stability, the catalyst may be operated at less severe conditions for a longer period of time with fewer periodic regeneration cycles and no loss of acetylene conversion. In addition, the product effluent has higher purity and requires less intense downstream purification processing. Larger diameter catalysts are expected to provide the same advantages as discussed above when at least 50 and preferably 70 weight percent of the copper metal and the activator metals are dispersed on the outer 200 µm of the catalyst support.

The evaporative impregnation technique may incorporate may incorporate copper and the activator metals on the catalytic support such that at least 50 weight percent of the metals are located in the outer 200 µm layer of the support, preferably at least 70 weight percent of the metals are located in the outer 200 µm layer of the support, and more preferably at least 80 weight percent of the metals are located in the outer 200 µm layer of the support. It is most preferred that at least 88 weight percent of the metals are located on the outer 200 µm layer of the support. "Layer" is meant to describe a stratum of substantially uniform thickness, and "outer" is meant to define the exterior layer of the support. In general terms. Surface impregnation can be carried out using metal complexes that have a high affinity for the support surface or complexes which are bulky in nature or by spray impregnation techniques in which the volume of the impregnating solution is less than that required to fill the pore volume. Techniques for surface impregnation are described in US-A-3,259,454; US-A-3,259,589; US-A-3,388,077, Lee, S.; Aris, R. Catal. Rev.-Sci: Eng. 1985, 27(2), 207-340; Komiyama, M. CataL Rev.-Sci. Eng. 1985, 27(2), 341-372; and Dougherty, R. C.; Verykios, X. E. Catal. Rev.-Sci. Eng. 1987, 29(1), 101-150.

Selective hydrogenation of the acetylenes contacts the hydrocarbons with the above-described catalytic composite in a fixed bed system. A heated reactant mixture enters a single bed, or multiple sub-beds of the fixed bed system. Heating means between the individual beds may maintain the reactants at the desired temperature. The fixed bed system may operate in a swing bed mode, with one sub-bed on-line and receiving the reactant mixture while another sub-bed is off-line. The off-line sub-bed may be undergoing regenerated, or may have completed regeneration and is ready for use. As the reactant mixture contacts the catalytic composite, the acetylenes are hydrogenated leaving the effluent stream essentially acetylene-free. Examples of the selective hydrogenation reactions include hydrogenating vinyl acetylene to form 1,3-butadiene, hydrogenating ethyl acetylene to form 1-butene, and hydrogenating methyl acetylene to form propylene. The amount of residual acetylenes expected in the reactor effluent is typically less than 15 wt-ppm. Conditions for the selective hydrogenation of C₄-acetylenes include a temperature in the range of 20°C to 80°C, pressures in the range of from 1500 kPa (15 bars) to 5,000 kPa (50 bars) and liquid hourly space velocities in the range of from 0.5 to 10 hr⁻¹. Hydrogen is also added at a hydrogen to acetylene ratio of from 1.0 to 5.0.

### EXAMPLE 1

The specific embodiment involves a process for selectively hydrogenating C₄-acetylenes in a liquid hydrocarbon stream containing largely butadiene comprising contacting hydrogen and the hydrocarbon stream with a catalytic composite which is an inorganic oxide support having dispersed thereon finely divided copper metal and optionally an activator metal selected from the group consisting of nickel, cobalt, platinum, palladium, manganese, and a combination thereof where the catalytic composite is spherical and has an average diameter of up to 800 µm (1/32 inch).

Alumina spheres were prepared by the oil drop method. Dissolving aluminum in hydrochloric acid formed an aluminum hydrosol. Added hexamethylene tetraamine to the hydrosol gelled the mixture into spheres when dispersing droplets into an oil bath maintained at 93°C. The droplets remained in the oil bath until they set and formed hydrogel spheres. The spheres were removed from the hot oil, pressure aged at 130°C, washed with dilute ammonium hydroxide solution, dried to 260°C and calcined at 640°C for 1.5 hours to give gamma alumina spheres having an average diameter of 1600 µm (1/16 inch).

The metal incorporation was performed using the evaporative impregnation technique. Dissolving copper nitrate, nickel nitrate, cobalt nitrate, and manganese nitrate in water prepared the impregnation solution. The resultant solution was then added to a rotary evaporator loaded with the gamma alumina spheres. After cold rolling the mixture for 1 hour steam was introduced to the outer jacket to evaporate the excess water. The metal impregnated catalyst was dried at 210°C for 1 hour and calcined at 400°C for 2 hours. The above process was repeated three times to give three reference catalysts having 1600 µm (1/16 inch) diameter spheres, identified as Reference 1, Reference 2, and Reference 3.

### EXAMPLE 2

Again, alumina spheres were prepared by the well known oil drop method involving forming an aluminum hydrosol by dissolving aluminum in hydrochloric acid. To this hydrosol, where was added hexamethylene tetraamine to gel the mixture into spheres when dispersed into droplets into an oil bath maintained at 93°C. The droplets remained in the oil bath until they set and formed hydrogel microspheres. After the microspheres were removed from the hot oil, they were pressure aged at 120°C and washed with dilute ammonium hydroxide solution, dried at 205°C and calcined at 640°C for 1.5 hours to give gamma alumina microspheres having an average diameter of 800 µm (1/32 inch).

The metal incorporation was performed in the manner described for example 1. The microspherical catalyst was analyzed and compared to the reference catalysts prepared in Example 1. Table 1 shows the results of the microspherical catalyst analysis as compared to the typical result for the three reference catalysts with all concentration units in weight percent of the catalytic composite.

**TABLE 1**

| | 800 µm (1/32 inch) | 1600 µm (1/16 inch) |
|---|---|---|
| copper, wt. % | 7.1 | 7.6 |
| nickel, wt. % | 0.2 | 0.19 |
| cobalt, wt. % | 0.10 | 0.10 |
| manganese, wt. % | 0.15 | 0.14 |
| average bulk density in g/cc | 0.73 | 0.80 |
| BET surface area, m²/g | 179 | 182 |

### EXAMPLE 3

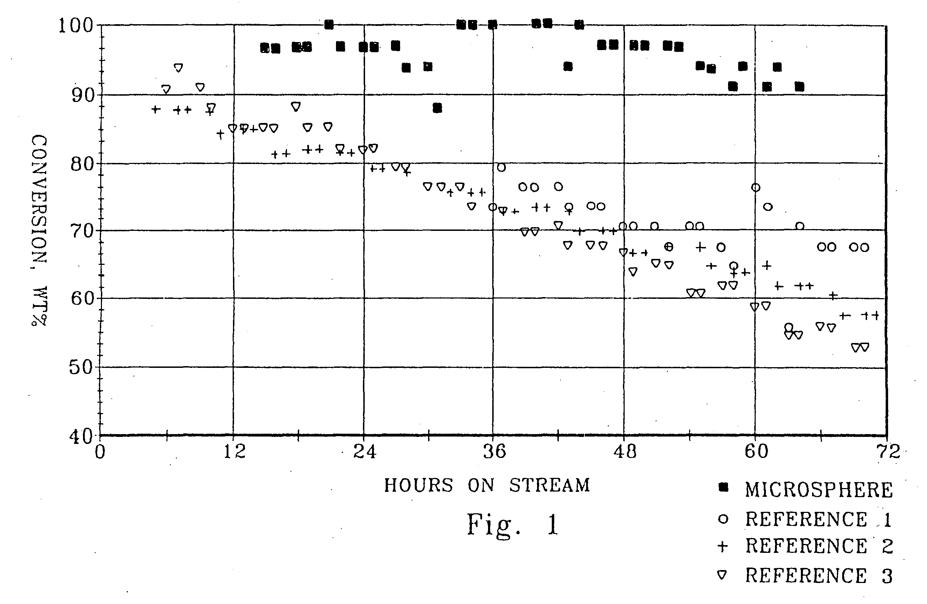

The catalysts prepared in Examples 1 and 2 were evaluated in a selective hydrogenation process with the results demonstrating the enhanced stability and selectivity of the microspherical catalyst of Example 2 as compared to Reference catalysts 1, 2, and 3 of Example 1. A reactor was loaded with 16 g of the microspherical catalyst prepared in Example 2 and heated to an inlet temperature of 60°C. A crude C₄ hydrocarbon stream from a naphtha cracker complex and containing 38 wt. % 1,3-butadiene and 0.35 weight percent of vinyl acetylene and 0.13 wt. % ethylacetylene was introduced to the reactor at an acetylene weight hourly space velocity of 0.15. The hydrogen to acetylene molar ratio was 2.1. The effluent was analyzed by gas chromatography and the resulting data is provided in Figures 1-6. The experiment was repeated three more times at the same conditions to test Reference catalysts 1, 2, and 3 of Example 1. The results of all analyses are provided in Figures 1-6.

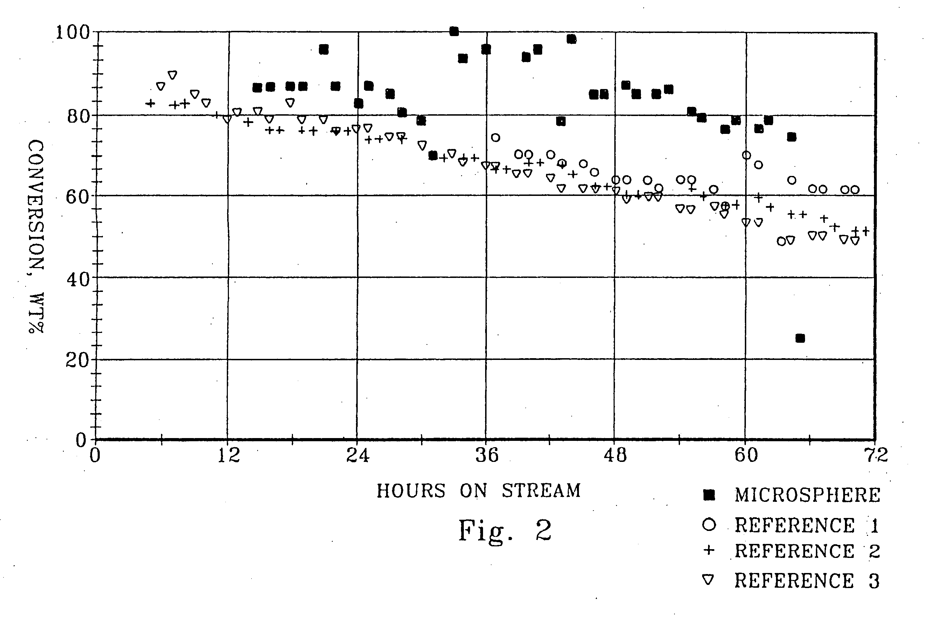

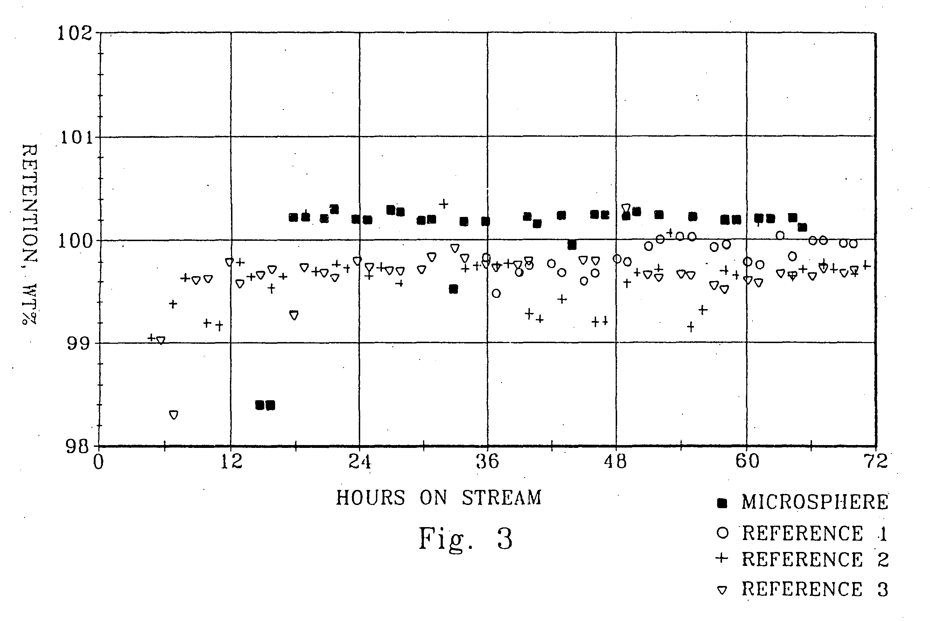

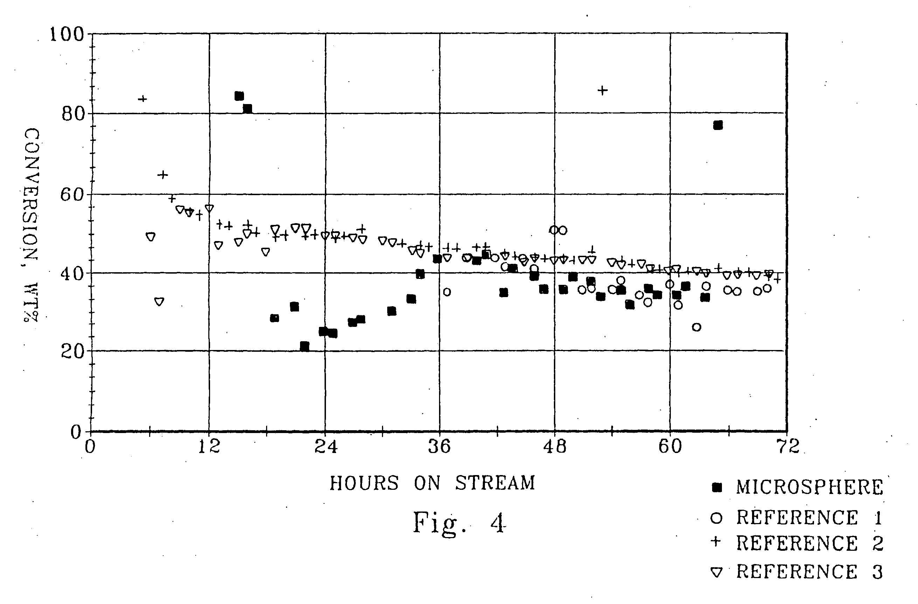

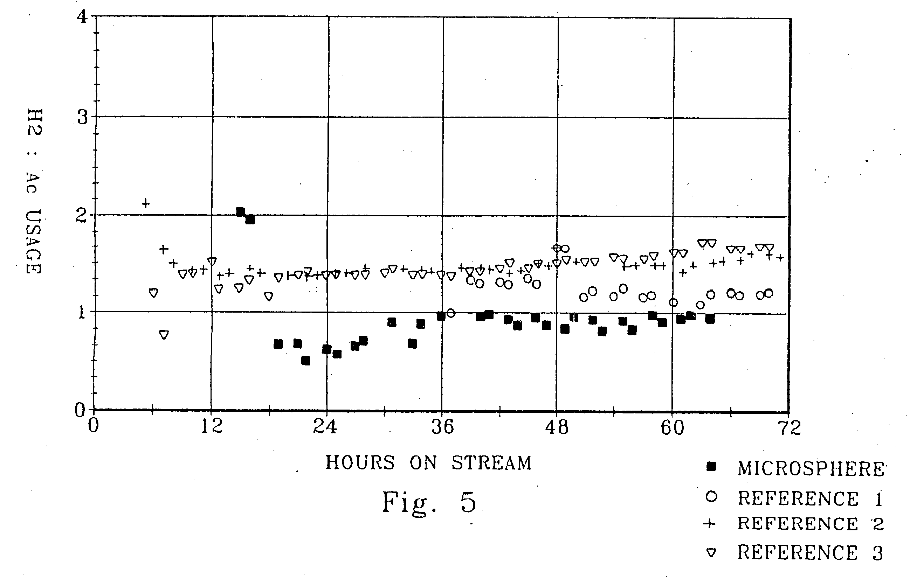

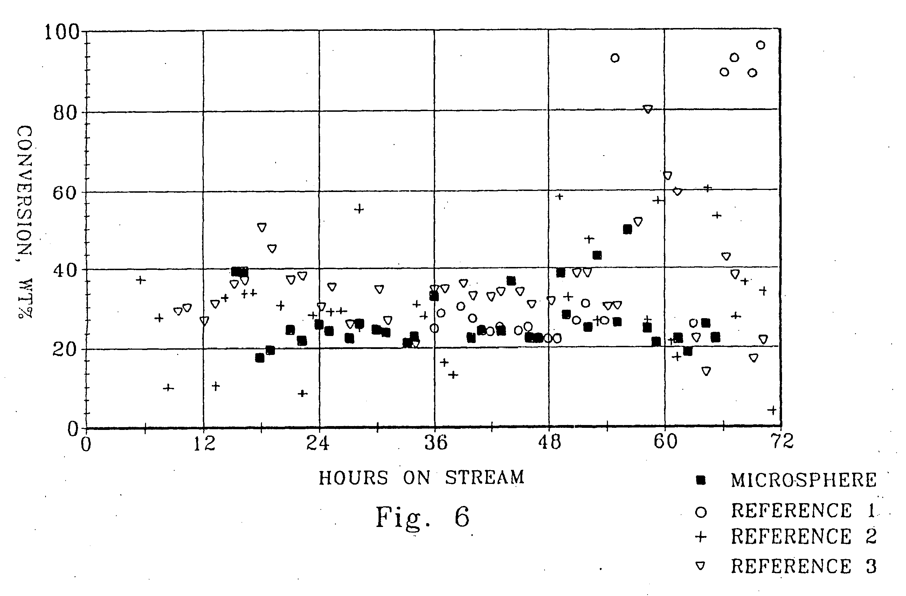

Figure 1 shows the weight percent conversion of vinyl acetylene that occurred over time during each of the experiments. The data clearly shows the increased stability of the microspherical catalyst over time as compared to the reference catalysts. The microspherical catalyst of Example 2 remained at greater than 90 weight percent conversion of vinyl acetylene for 70 hours on stream, while the reference catalysts of Example 1 showed less than 80 weight percent conversion at 70 hours on stream. Similarly, Figure 2 shows the microspherical catalyst of Example 2 remained at greater than 78 weight percent conversion of total acetylenes at 70 hours on stream, while the reference catalysts of Example 1 showed less than 75 weight percent conversion total acetylenes at 70 hours on stream. The enhanced selectivity of the microspherical catalyst is demonstrated in Figure 3 which shows higher butadiene retention (weight percent of butadiene in the effluent divided by weight percent butadiene in the feed) with the microspherical catalyst; in Figure 4 which shows overall less hydrogen conversion when using the microspherical catalyst; and in Figure 5 which shows lower hydrogen to acetylene usage ratio indicating that less hydrogen is being consumed through hydrogenation of butadiene when using the microspherical catalyst. Figure 6 indicates that the selectivity for polymeric byproducts, or green oil, is less when using the microspherical catalyst of Example 1.

## Claims

1. A process for selectively hydrogenating C4-acetylenes in a liquid hydrocarbon stream containing largely butadiene comprising contacting hydrogen and the hydrocarbon stream with a catalytic composite comprising an inorganic oxide support having dispersed thereon finely divided copper metal and an activator metal selected from the group consisting of nickel, cobalt, platinum, palladium, manganese, and a combination thereof said catalytic composite having an average effective diameter of up to 800 µm (1/32 inch).

2. The process of claim 1 wherein at least 70 weight percent and preferably at least 80 weight percent of said copper metal and said activator metal are dispersed on the outer 200 µm layer of the support.

3. The process of Claim 1 or 2 wherein the catalytic composite is of a shape selected from the group consisting of a sphere and an extrudate.

4. The process of any of Claim 3 wherein the selective hydrogenation conditions include a temperature of 20°C to 80°C, a pressure of from 1500 kPa (15 bars) to 5,000 kPa (50 bars), a liquid hourly space velocity of from 0.5 to 10hr^{-1,} and a hydrogen to acetylene ratio of 1.0 to 5.0.

5. The process of any of Claim 3 wherein said copper metal is present in an amount ranging from 5 to 15 weight percent of the catalytic composite and said activator metal is present in an amount ranging of from 0.1 to 1 weight percent of the catalytic composite.
